# NEW EUROPEAN PATENT SPECIFICATION

(11) **EP 0 216 846 B2**
(45) Date of publication and mention of the opposition decision: **26.04.1995**
(45) Mention of the grant of the patent: 10.01.1990
(21) Application number: 86901978.6
(22) Date of filing: 01.04.1986
(51) Int. Cl.: C12N 15/00, C12N 5/00, C12P 21/00

(54) **TRANSFORMED MYELOMA CELL-LINE AND A PROCESS FOR THE EXPRESSION OF A GENE CODING FOR A EUKARYOTIC POLYPEPTIDE EMPLOYING SAME**
TRANSFORMIERTE MYELOMA-ZELL-LINIE UND DIESELBE VERWENDENDES VERFAHREN ZUR EXPRESSION EINES GENS, DAS EIN EUKARYONTISCHES POLYPEPTID KODIERT
LIGNEE CELLULAIRE DE MYELOMES TRANSFORMEE ET PROCEDE D'EXPRESSION D'UN GENE CODANT UN POLYPEPTIDE EUCARYOTIQUE EMPLOYANT CETTE LIGNEE

(30) Priority: 01.04.1985 GB 8508442; 03.09.1985 GB 8521815
(43) Date of publication of application: 08.04.1987
(73) Proprietor: CELLTECH LIMITED, Slough Berkshire SL1 4DY (GB)
(72) Inventor: KENTEN, John, Henry, Gaithersburg, Maryland 20879 (US); BOSS, Michael, Alan, Middlesex HA4 7HN (GB)
(74) Representative: Votier, Sidney David
(86) International application number: GB8600187
(87) International publication number: WO8605807

(56) References cited:
- EP-A- 0 043 718
- Nature, vol. 276, 16 November 1978, M. Shulman et al.: "A better cell line for making hybridomas secreting specific antibodies", pages 269-270
- Gene, vol. 33, no. 2, February 1985, A.Kudo et al.: "A cloned human immunoglobulin heavy chain gene with a novel direct-repeat sequence in 5' flanking regions", pages 181-189
- Proceedings of the National Academy of Sciences USA, vol. 80, February 1983; V.T. Oi et al.: "Immunoglobulin gene expression in transformed lymphoid cells", pages 825-829
- Advances in Cancer Research, vol. 43, pages 205-239, 1985, Academic Press; K. Moelling
- PNAS USA, vol. 80, Febr. 1983, pp. 825-829, V.T. Oi et al.
- Science, vol. 209, 19 Sept. 1980, pp. 1422-1427, Mulligan & Berg
- Science, vol. 222, 1983, p. 809, Palmiter et al.
- Declaration of Dr. C.R. Bebbington
- Declaration of Dr. M.S. Neuberger

## Description

### Field of the Invention

This invention relates to the field of recombinant DNA biotechnology. In particular, it relates to transformed myeloma cell-line and to a process for the expression of a gene coding for a eukaryotic polypeptide in myeloma cells.

### Background to the Invention

In recent years, advances in biotechnology have led to an ability to produce eukaryotic polypeptides on an industrial scale by the expression of appropriate genes in the cells of host cell-lines. The commercial success of such processes depends upon the efficiency with which a particular gene can be expressed in the cells of a given host cell-line. There is a continued need for expression systems exhibiting high expression efficiency.

Early expression experiments were conducted using bacterial cell-lines. However, the disparity between such cellular environments and the normal environment of eukaryotic polypeptides leads to some undesirable effects and generally to a low product output. These effects have been, to some extent, ameliorated by the use of eukaryotic cell-lines, particularly mammalian cell-lines, as hosts. A variety of mammalian cell-line expression systems are known (see for example United States patent specification 4419446 (Howley, *et al*) which describes the transformation of mouse fibroblast cells with a vector including a gene coding for human insulin).

Studies relating to the expression of immunoglobulin genes have involved the use of recombinant DNA techniques to clone the relevant genes and to transform mammalian cells with the cloned genes. (Morrison, S. L. and Oi, V. T. Ann Rev. Immunol. (1984) *2* 239―256). Myeloma cell-lines have been used as hosts and various vectors have been produced to transform myeloma cell-lines. These vectors in general carry an immunoglobulin promoter and gene. The vectors also include selectable markers to allow selection of host myeloma cells which have been successfully transformed. The markers comprise viral promoters driving expression of prokaryotic genes coding for non-secreted products which confer antibiotic resistance on transformed host cells. The products, which have a catalytic inactivating effect upon antibiotics, are prodcued at low levels.

These studies have shown that myeloma cell-lines exhibit a very specialised function in the expression of immunoglobulin genes. It has been shown that chimaeric mouse/human immunoglobulins can be produced in myeloma cells (Morrison, S. L. *et al* PNAS USA (1984) *81* 6851-6588; Neuberger, M. S. Nature (1985) *314* 268―270; Boulianne, G. L. Nature (1984) *312* 643―646) and also that chimaeric immunoglobulin/enzyme and immunoglobulin/antigen polypeptides can be produced in myeloma cells. In all cases, an immunoglobulin promoter is used to direct expression of the desired product which comprises, at least in part, an immunoglobulin.

It has now been surprisingly discovered that it is possible to express eukaryotic genes at high levels in myeloma cell-lines from viral promoters or a mouse metallothionein promoter. This result is surprising in view of the specialised nature of myeloma cell and the known dependence upon cell type of the expression of eukaryotic genes.

### Summary of the Invention

According to a first aspect of the invention there is provided a myeloma cell-line transformed with a vector including a gene coding for a eukaryotic polypeptide and a viral promoter or a mouse metallothionein promoter, such that expression occurs of the gene coding for the eukaryotic polypeptide directed by the viral promoter or a mouse metallothionein promoter. Myeloma cell lines according to the present invention wherein expression occurs of the gene coding for the eucaryotic polypeptide under the direction of a viral promoter and wherein the eucaryotic polypeptide is produced at a level of 1 mg/l or less are hereby disclaimed.

According to a second aspect of the present invention there is provided a process for preparing a eukaryotic polypeptide comprising culturing myeloma cells transformed with a vector including a gene coding for the eukaryotic polypeptide and a viral promoter or a mouse metallothionein promoter, such that when the vector is transformed into myeloma cells, expression occurs of the gene coding for the eukaryotic peptide, directed by the viral promoter of the mouse metallothionein promoter. Processes according to the present invention wherein expression occurs of the gene coding for the eucaryotic polypeptide under the direction of a viral promoter and wherein the eucaryotic polypeptide is produced at a level of 1 mg/l or less are hereby disclaimed.

The vector, which may be a plasmid and which may be an episomal vector, but is preferably an integration vector, includes a selectable marker, such as a resistance marker. The resistance marker may be placed near to the site of insertion of the heterologous coding sequence and is preferably under the control of a separate promoter, for example, an SV40 promoter. The resistance marker may be replaced with or supplemented with an amplifiable gene such as the gene coding for dihydrofolate reductase (dhfr).

The promoter is preferably a viral promoter, most preferably derived from a retrovirus, for example, a long terminal repeat (LTR) derived from such a retrovirus. Examples of suitable promoters are a Simian virus 40 (SV40) promoter preferably the late SV40 promoter and the Rous sarcoma virus long terminal repeat (RSV LTR). The RSV LTR is a DNA sequence which has previously been described as a strong promoter when introduced into a variety of eukaryotic cells such as fibroblast cell-lines (Gorman, *et al* (1982) PNAS *79* 6777―6781). However, due to he specialised nature of myeloma cells, it is entirely unexpected that the RSV LTR proves effective in a myeloma cellular environment.

Alternatively the promoter may be non-viral, such as the mouse metallothionein promoter.

The term "myeloma" as used herein encompasses mammalian myeloma cells and cells derived therefrom by fusion, such as hybridoma type cell-lines.

The eukaryotic polypeptide may be a mammalian polypeptide such as an enzyme (for exmaple, chymosin), an enzyme inhibitor, a hormone (for example, growth hormone), a lymphokine (for example, an interferon), or an immunoglobulin or a fragment thereof (for example a fab fragment).

The eukaryotic polypeptide may be a fusion polypeptide comprising at least in part a eukaryotic polypeptide. The eukaryotic polypeptide may be in a naturally occurring form or in the form of a functionally equivalent derivative. The eukaryotic polypeptide may include a signal sequence allowing export of the polypeptide from the host myeloma cell.

In the embodiment described below by way of example, the eukaryotic polypeptide is tissue plasminogen activator (tPA), an especially preferred product.

Particular expression vectors of the invention are designated p6, p3.16, pRSV3, pZAP7, pAC1, pAC2, pAC5 p6GD, p6gpt, pR5D3, pAC6 and pPRI, the construction of which from available materials is described in detail below.

The myeloma cell-line may be a rat or mouse myeloma or hybridoma cell-line, such as the rat YB2/3.0 Ag20 hybridoma cell-line, the mouse NSO hybridoma cell-line or the mouse SP2-0Ag hydriboma cell-line.

We have discovered that certain combinations of myeloma cell-lines with vectors carrying specific promoters exhibit advantageous properties. Preferably when the cell-line is a rat cell-line, such as the rat YB/3.0 Ag20 hybridoma cell-line, the promoter used is the RSV LTR. Preferably where the cell-line is a mouse cell-line, such as the mouse SP2-OAg hybridoma, the mouse NSO hybridoma cell-line or an NSO-derived hybridoma cell-line, the promoter used is the SV40 late promoter.

Where the eukaryotic polypeptide includes the relevant signal sequences (at least in the initially translated product) export of the polypeptide cocurs to the culture supernatant, allowing an improved process which does not require cell disruption to harvest the product.

The process for preparing a eukaryotic polypeptide may comprise isolating the polypeptide from the culture supernatant of a culture of myeloma cells transformed with a vector of the invention such that expression of the gene and secretion of the polypeptide product occurs.

Rat hybridoma cell-line YB2/3.0 Ag20 is described in British patent specification 2079313 and is on deposit at the American Type Culture Collection (as YB2/O or YB2/3HL. P2. G11. 16Ag.20) under Accession Number CRL 1662 (Date of deposition earlier than 1st June, 1985).

Mouse hybridoma cell-line SP2-OAg14 is on deposit at the American Culture Collection under Accession Number CRL 1581 (Date of deposition earlier than 1st June, 1985).

Mouse hybridoma cell-line P3/NS1/1 Ag4.0 (the NS1 cell-line) is on deposit at the American Culture Collection under Accession Number T1B18 (Date of deposition 10th November, 1981).

### Brief Description of Drawings

The invention is now described with reference to the accompanying drawings in which:
Figure 1 shows restriction maps of plasmids pSV2 B globin, pSV3 Bne, pSV3 MMne, p6, p3.16, pRSV3, pZAP7, pAC1, pAC2, pAC5, pAC6 and pPR1 (The direction of transcription of the tPA and BPV genes is denoted by arrows. The positions of the restriction enzyme sites for *Eco*RI, *Bam*HI and *Sal*I are denoted E, B and S respectively. The origin of the various sequences are denoted: 
BPV
pBR322 derived DNA;
mouse metallothionein promoter;
LTR promoters from either mouse Moloney leukaemia virus (pACI, pAC3) or Rous Sarcoma
virus (pAC4, pAC5, pAC6 and pPR1);
tPA cDNA gene;
SV40 polyA site;
SV40 late promoter;
selection gene;
SV40 early promoter;
SV40 sequences).
Figure 2 comprises photographs of fibrin agarose plates containing cells (SP2/O―Ag14) transfected with identical plasmid constructs, except for promoter fragments 5' of the tPA gene, at times 24 hours and 48 hours from the start of transfection, and
Figure 3 is a graph showing profiles for growth and tPA synthesis by pRI 1/10 cells in an air lift fermenter (Viable cells △; tPA determined by ELISA ●; tPA determined by fibrin agar assay ○).

### Description of Specific Embodiments

### 1. Vector Construction

### 1.1 Tissue plasminogen activator (tPA) cDNA expression constructs

The methods used in these constructions are as described in detail by Maniatis *et al* (1982). The tPA specific DNA sequence used in this study were derived from two cDNA clones isolated from a Bowes melanoma library (Harris *et al* 1986). Plasmid ptPA A3 was constructed by adding HindIII linkers to a 1200 base pair (bp) fragment (nucleotides 9 to 1197, Pennica *et al,* 1983) of tPA cDNA. The cDNA was then cloned into pAT153 at the unique HindIII site. Plasmid ptPA21 was constructed by cloning a Bgl 11 cDNA fragment (nucleotides 187 to 2166) into a plasmid with a unique BglII site. A full-length tPA cDNA gene was made by cloning the BglII fragment from ptPA21 into the BglII site of ptPA A3. The resultant plasmid ptPA 3/21 contains 76 bp of 5' non-coding sequence, the complete tPA coding region, 393 bp of 3' non-coding sequence, and a repeat segment of tPA coding region (nucleotides 187 to 1197), all on a single HindIII fragment. A second plasmid p81/11 was also constructed in which this HindIII fragment was inverted relative to pAT153 sequences.

Plasmaid p81/11 was digested with BalI and the BalI fragment containing 116 nucleotides of 3' non-coding sequence, the repeated segment of tPA coding region, and some pAT 153 sequences were removed leaving plasmid pBSI. Single BamHI and SalI restriction enzyme sites were re-introduced into pBSI by ligation of the 275 bp BamHI/SalI fragment from pAT 153, whose cohesive termini had been filled in with DNA polymerase, into the BalI site. Depending upon the orientation in which the fragment was inserted, either a SalI site (pBS17) or a BaMHI site (pBS18) was reformed at the 3' end of the tPA gene. Plasmid pBS18 was the precursor for all tPA cDNA expression constructs.

### 1.2 Mouse Metallothionein (MMT―I) Promoter

Clone 28 (Dr. D. Hamer; National Institute of Health, Bethesda, Maryland, U.S.A.) has the entire SV40 genome cloned into a BamHI site and a 3.8Kb *Eco*RI fragment containing the mouse metallothionein I (MMT―I) gene (Hamer and Walling, 1982) cloned into an *Eco*RI site. This plasmid provided the MMT―I promoter (MMT) in tPA expression constructs. The unique BglI site, 4 bp upstream of the translation start point (Glanville, *et al*, 1981), was converted to a HindIII site using the oligonucleotide CCAAGCTTGG.

A 2.0 Kbp HindIII fragment derived from clone 28 containing the MMT promoter was cloned into the unique HindIII site of pBS18 resulting in the formation of a transcriptional fusion between the MMT promoter and tPA coding sequences. This plasmid was called pBS18M3. A BamHI/BclI fragment of 243 bp derived from clone 28 and containing the SV40 polyadenylation site was inserted into the BamHI site of pBS18M3. The polyadenylation site was orientated in the early to late direction thus recreating a BamHI site distal to the tPA gene. This plasmid was denoted p3.16.

### 1.3 Rous Sarcoma Virus and Moloney Murine Leukaemia Virus Long Terminal Repeats

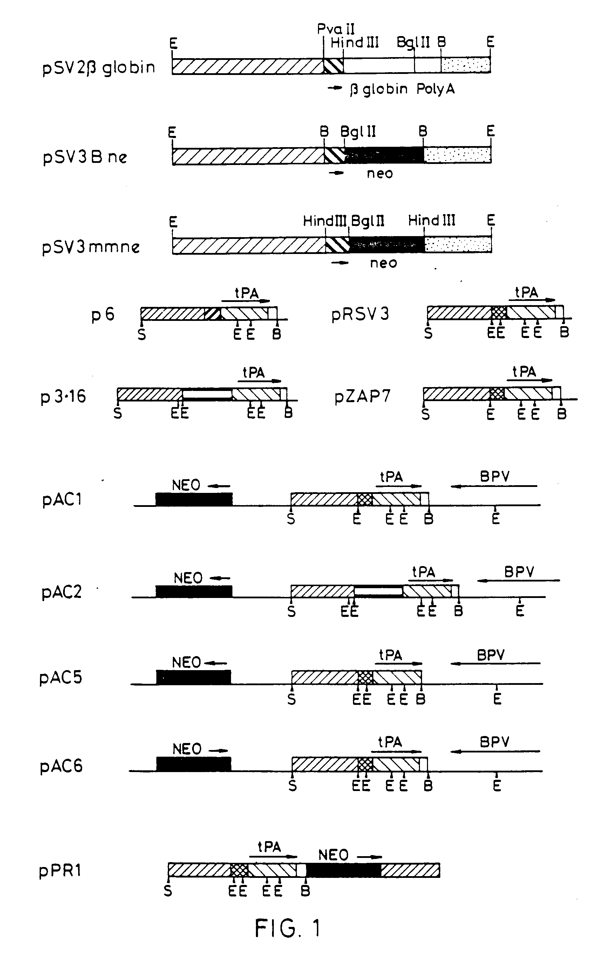

The long terminal repeats (LTR) of the Rous sarcoma virus (RSV) and Moloney murine leukaemia virus (Mo MLV) were isolated as ClaI/HindIII fragments from pRSVcat (Gorman *et al*. 1982) and p836 (Hoffmann *et al*, 1982), respectively. In the former case, a MbOII site was converted to a ClaI site using the oligonucleotide GGATCGATCC and in the latter case, a SmaI site was converted to a HindIII site using the oligonucleotide CCAAGCTTGG. The fragments were individually cloned into p3.16, previously cut with ClaI and HindIII, so that the MMT promoter could be replaced by a retrovirus LTR promoter. The resultant plasmids were called pRSV3 (containing the RSV LTR) and pZAP7 (containing the MoMLV LTR). (see Figure 1).

### 1.4 SV40 Late Promoter

The plasmid containing the SV40 late promoter driving tPA was constructed using p3.16 as the basic vector. The SV40 late promoter was isolated from pSV2 neo by PvuII digestion, followed by addition of HindIII linkers, and subsequent HindIII digestion. The SV40 late promoter fragment so generated was purified by gel electrophoresis and used to replace the HindIII promoter fragment of MMT from plasmid p3.16. The plasmid generated was denoted p6. (Figure 1).

### 1.5 Comparative Example - Immunoglobulin Promoter/Enhancer

A plasmid containing an immunoglobulin promoter/enhancer combination was constructed using pAT153 as the basic vector (Twigg and Sherratt, 1980). The EcoRI-BamHI region in pAT153 was replaced by the Ig heavy chain enhancer (Ig en) on a XbaI-EcoRI fragment, (Gillies *et al*, 1983) converted to an EcoRI fragment by addition of EcoRI linkers. The Ig en was cloned in the sense orientation relative to an Ig heavy chain promoter (Ig Pro) isolated as a 1.3kb EcoRI-NcoI fragment. This promoter fragment is derived from phage clone VNPB-186; (Bothwell *et al*, 1981) with its NcoI site filled in by polymerase and converted to BglII site by linker addition, resulting in generation of vector pMI205. The tPA gene was introduced into this vector as a BamHI fragment. This was isolated from pBS18 as a BamHI-HindIII fragment followed by addition of BamHI linkers. The tPA BamHI fragment was introduced into the BglII site of pMI205 creating pMI205tPA.

The five plasmids p3.16, pRSV3, pZAP7, p6 and pMI205 (Figure 1) were the direct precursors of the tPA expression constructs, and also the plasmids used in transient assays for tPA production.

### 1.6 Selection Markers and other Functional Fragments

The genes responsible for conferring dominant selection were derived from pSV2 neo (Southern and Berg, 1982), pSV2dhfr (Subramani *et al*, 1981) and pSV2gpt (Mulligan and Berg, 1981).

The resistance to antibiotic G418 conferred by the transcription unit in pSV2 neo ('neo'), was used in the majority of the described constructs. In order for the 'neo' transcription unit to be used it was recloned to be contained within both a BamHI and also a HindIII fragment.

The starting point for these vector constructs was pSV2 βglobin (Figure 1), (Southern and Berg, 1982). The vector's HindIII site was converted into a BglII site followed by removal of the resultant BglII fragment containing βglobin. The resultant plasmid, pSV2 BglII formed the basis of the next step-conversion of the PvuII site into a HindIII site generating plasmid pSV3M.

The introduction of the 'neo' gene into pSV3M was via replacement of the BglII-BamHI poly A-splice gene fragment by a BglII-BaMHI fragment isolated from pSV2 neo. The resultant plasmid, pSV3Mne, now contained the SV40 early promoter driving the expression of the 'neo' gene without a poly A sequence, contained within a HindIII-BamHI fragment. The HindII and BamHI sites of pSV3Mne were separately converted into BamHI and HindIII sites respectively resulting in the generation of plasmids pSV3Bne and pSV3MMne (Figure 1) respectively.

In order to produce a suitable gene fragment for the use of the xanthine-guanine phosphoribosyltransferase (gpt) gene from pSV2gpt (Mulligan and Berg, 1981), conferring dominant selection in the presence of mycophenolic acid and xanthine (Morrison and Oi, 1984), we replaced the BglII-BamHI fragment of pSV3Mne containing the 'neo' gene with the BglII-BamHI fragment of pSV2 gpt containing the gpt gene. The resultant plasmid pSV3 gpt contains the SV40 early promoter driving expression of gpt with a poly A-splice from SV40 at the 3' end on a HindIII-BamHI fragment. This vector's HindIII site was then converted to a BamHI site, producing plasmid pSV3 Bgpt, providing a suitable BamHI fragment for use in the described vectors.

To enable a study of the utility of amplification within myeloma cells, we made use of the mouse dihydrofolate reductase (dhfr) cDNA gene as contained in pSV2 dhfr (Subramani *et al*, 1981).

In order to produce a gene fragment of the SV40 early promoter and dhfr suitable for our vectors, we converted the PvuII site of pSV2 dhfr to a BamHI site by linker addition, generating plasmid pDB. pDB contains a BamHI fragment containing the SV40 early promoter driving the dhfr gene with the SV40 poly A-splice sequence at the 3' end.

Plasmid pBMTHI (Dr. G. N. Pavlakis; National Cancer Institute; Frederick, Maryland) contains the entire BPV-1 genome and the complete MMT―I gene on a BamHI/SalI fragment. A second plasmid, pBMT13, was constructed from pBMTHI in which the BamHI site was converted to a SalI site and the SalI site was converted to a BamHI site.

Initial vectors were based on BPV vectors in order to examine the potential value of these. The studies with pBMT13 demonstrated that heavy metal selection with myeloma cells was not useful. This raised the need for another selection system within BPV. In order to construct BPV vectors conferring dominant selection in myeloma cells, we introduced the 'neo' gene cassette from pSV3MMne isolated as a HindIII fragment enabling the direct replacement of the MMT―I gene in pBMT13. This replacement of MMT―I gene resulted in two new BPV-based plasmid vectors, p2012SneR and p2012SneL, containing the SV40 early promoter driving the 'neo' gene as a HindIII insert, in both orientations relative to BPV. These BPV vectors formed the basis of the vectors tested in myeloma cells using the tPA plasmids described above p6, p316, pRSV3 and pZAP7. The resultant plasmids pAC1, pAC2, pAC5, and pAC6 were generated by replacing the BamHI-SalI pAT153 sequence with BamHI-SalI fragments from p6, p316, pRSV3 and pZAP7.

Further constructs were made without the use of BPV. In these later constructs, use was made of the BamHI fragments from pSV3ne, pSV3 Bgpt and pDB conferring a dominant selectable marker on the plasmids.

In order to use pRSV3 for generation of stable cell lines, in the absence of a BPV vector conferring selection, a selection marker was introduced into the BamHI site. The selection cassette used was the BamHI fragment from pSV3Bne, producing plasmids pPRI and pPRII containing the BamHI fragment in both orientations. In addition to the 'neo' gene, the BamHI selection cassette from pSV3 Bgpt and pDB were also introduced at the BamHI site, resulting in the production of pRSG containing the gpt gene and pRSD3 containing three or more dhfr gene cassettes.

To make use of the SV40 late promoter driving tPA^{·} expression, in the absence of BPV, similar constructions as above were made. The BamHI gpt gene cassette from pSV3 Bgpt was introduced producing p6 gpt. The utility of the p6 gpt vector was further enhanced by the introduction into partial BamHI-digested p6 gpt the dhfr BamHI expression cassette, giving rise to p6GD.

### 2. Transfections

The cell lines used for transfections were SP2-OAg 14 (Shulman *et al*, 1978); NSO, this is a subline of P3/NS1/1 Ag4.1 (Kohler *et al*, 1976) that does not express the intracellular light chains (M. Clark, B. W. Wright and C. Milstein); 001 a BALB/c X NSO hybridoma (Sherwood, M. unpublished) and YB2/3.0 Ag20 (U.K. Patent GB2079313). These cell lines were maintained in Dulbecco modified Eagle's medium (DMEM, Gibco Ltd., U.K.) with penicillin (50U ml), streptomycin (50 »g/Ml), 1 mM pyruvate, 2 mML-glutamine and heat inactivated foetal calf serum (10%).

In order to introduce DNA into myeloma cells we have made use of the standard methods using DEAE-Dextran, CaPO4 and electroporation (Banerji *et al*, 1983; Graham and Van der Eb, 1973; Potter *et al*, 1984). The majority of our transfections were made using a modification to the DEAE-Dextran method making use of dimethyl sulphoxide (DMSO) stock (Lopata *et al*, 1984) and chloroquinine (Lathman and Magnusson, 1983) to improve transfections.

The cells, after transfections, were typically left for 24 hours before selection was introduced. The transfections with plasmid constructs containing the 'neo' gene cassette from either pSV3Bne or pSV3MMne were selected using the antibiotic G418 (Geneticin Gibco Ltd., U.K. [Schering Corp. U.S. patent specification 3959254, 3997403]) at a concentration of 1.4 mg/ml.

The plasmids containing the gpt gene cassette from pSV3 Bgpt were selected for by supplementing the culture medium with 200 »g/ml xanthine, 5 »g/ml mycophenolic acid (Gibco, U.K.) and 13.6 »g/ml of hypoxanthine.

The selection of plasmids containing dhfr gene cassette from pDB was carried out at 150 nM methotrexate (Page, 1985).

In the case of transfection with p6GD, cell lines were initially selected making use of the gpt gene cassette followed by the utilisation of the dhfr gene cassette for amplification.

The cells, after the introduction of the selection, were allowed to incubate again for a further 24―48 hours before being plated out into microtitre dishes for clone selection. These microtitre dishes were typically incubated for 2―3 weeks before the first clones appeared. The clones were allowed to grow up to saturation (turning the culture media phenol red to yellow) prior to assaying the supernatants or to expansion into 24 well tissue culture dishes. The cell lines from those transfections which demonstrated useful levels of tPA production (greater than 100 U/ml were stored as frozen stocks in liquid nitrogen after supplementing the culture medium with 10% DMSO. These cell lines were also recloned and selected high producers also stored as above.

### Quantitation of tPA protein

The tPA from cell lines was either assayed via fibrinolysis or by an enzyme linked immunosorbent assay (ELISA).

Active tPA protein in the medium was assayed using a fibrin-agarose plate assay as modified by Cederholm-Williams. LGT agarose, 20 mg/ml, in 0.1 M Tris (pH 7.4), 0.15 M NaCl, and 2 mM CaCl₂ was melted and cooled to 55°C. An equal volume of fibrinogen, 2 mg/ml in 0.9% (w/v) NaCl, and human plasminogen to a final concentration of 10 »g/ml were added. Fibrin polymerisation was initiated by adding bovine thrombin to 0.12 units/ml. The mixture was poured on polyester sheets and allowed to set. Dilutions of samples and dilutions of a urokinase standard solution of known concentration (5 »l volume) were added to wells punched in the gel and the plates were incubated at 37°C for 17 to 20 hours in a humidified chamber. The diameters of the areas of lysis were measured. A standard curve was drawn from the urokinase data by plotting the log of the diameter squared against the log of the concentration. The amount of activity in the samples was determined from the standard curve. Within the 10 to 100 units/ml range, urokinase was shown to be a suitable standard for estimating the tPA activity.

Total tPA protein in culture medium from the transfected cell line was assayed using an ELISA. The ELISA was performed in Nunc Immuno assay plates (Nunc, Denmark) coated for 1 hour at 37°C with 2 »g/ml of a goat polyclonal anti tPA (Biopool, Sweden) in 0.05M sodium carbonate (pH 9.6). These plates were blocked for 1 hour at 37°C with 0.5% casein Hammerstem in 0.05M sodium carbonate (pH 9.6). These coated and blocked microtitre plates, and plates at other wash stages were washed with phosphate buffered saline, with 0.02% Tween 20. Samples for assay were diluted into sample buffer (0.1 M Tris-HCl pH 7.0, 150 mM NaCl, 0.5% casein and 0.02% Tween 20). The standard used in this assay was two chain tPA (Biopool, Sweden). Samples were introduced at 100 »l per well and incubated for 1 hour at room temperature with shaking. The plates were then washed and 1 »g/ml of MAC010, a mouse monoclonal antibody to tPA, in sample buffer added to each well (100 »l). These were incubated for 1 hour at room temperature with shaking. The plates, after incubation with the monoclonal antibody, were washed and 100 »l of goat anti-mouse IgG Fc specific peroxidase conjugate was added to each well and incubated for 1 hour at room temperature with shaking. The plates, after incubation with conjugate, were washed and then developed as described (Bos *et al*, 1981).

### Transient Assays of tPA Production in Myeloma Cells

In order to evaluate the ability of our plasmid constructs to direct the expression of eukaryotic gene products, we adapted the fibrin overlay assay for plasminogen activators into a rapidly sensitive and consequently powerful transient assay system (Jones *et al*, 1975; Kenten *et al*, 1986).

The plasmids used for these transient assays of tPA production were those described earlier namely:
p3.16 containing the mouse metallothionein promoter,
pRSV3 containing the RSV LTR,
pZAP7 containing the MoMLV LTR,
p6 containing the SV40 late promoter and
pMI205 tPA containing an immunoglobulin promoter/enhancer combination.

The transfections were carried out using the DEAE-Dextran method as described and the cells were put directly into the tPA assay described below.

For the tPA assay, transfected cells were scraped off the plate (about 5 × 10⁶) and 10% of the cells taken and washed twice in serum-free DMEM (Gibco), 1 mM pyruvate, 50 IU/ml penicillin, 50 ug/ml streptomycin (P/S), 2 mM L-glutamine (Gln). These cells were then suspended in 7 ml of 70% DMEM as above, supplemented with 10% (v/v) acid treated foetal calf serum (FCS); 30% (v/v) Hanks balanced salts (Gibco), supplemented with 2.5% low gelling temperature agarose (Sigma Ltd.) at 42°C. This suspension was then supplemented with 0.5 units of thrombin (from bovine plasma 500 U/ml, Sigma Ltd.). Finally, 1.5 mls of DMEM, 1 mM pyruvate, P/S, Gln, 10% (v/v) acid treated FCS, 30 mg/ml fibrinogen (from bovine blood, type I―S, Sigma Ltd.) was added and the mixture poured immediately into a 90 mm dish.

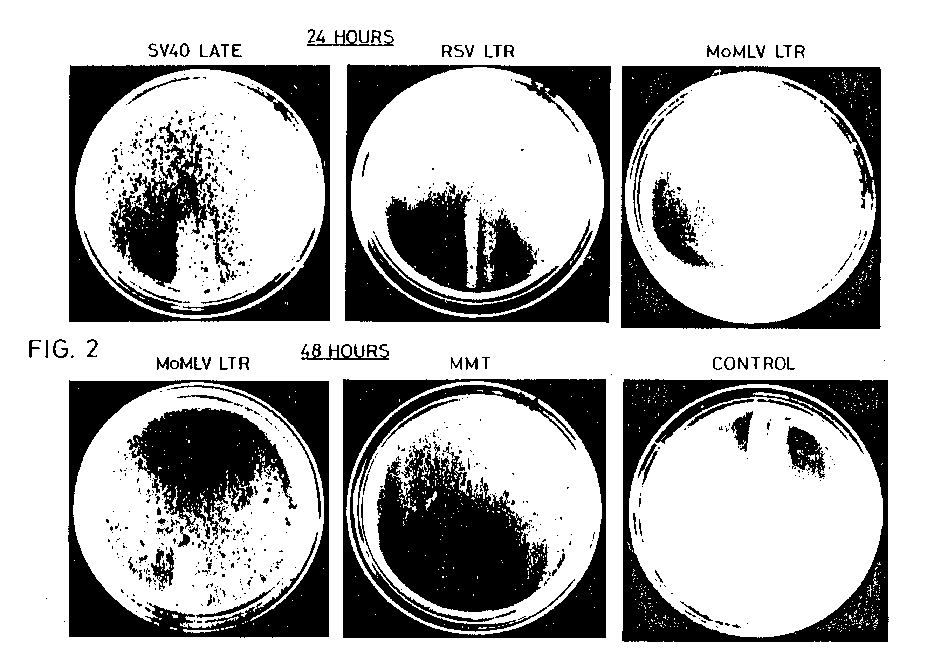

These dishes were incubated for times up to 48 hours in order to determine the level of tPA being produced from these transfections. The results were recorded as relative levels of fibrinolytic activity, Figure 2 shows a typical result.

The results from these transfections demonstrated that for YB2/3.0 Ag20 promoters were ranked: RSV LTR, SV40 late, MoMLV LTR and MMT in descending order of activity. Cell lines SP2/0―Ag14 and a BALB/c X NSO mouse hybridoma demonstrated a ranking of the promoters: SV40 late, RSV LTR, MoMLV LTR and MMT in descending order of activity.

We also tested one immunoglobulin promoter in combination with a partially characterised enhancer fragment as described in the vector constructs section above. This gave levels of tPA expression very similar to the MMT promoter in myeloma cell lines.

The results from these assays provide a useful framework on which to base an initial study of cell lines with integrated genes for expression. The transient assay only provides a measure of the promoter strength isolated from the normal gene environment (within the chromosome). Thus one may find differences between results from transient and stable cell-lines in terms of promoters preference.

### tPA Producing Cell Lines

The tPA producing cell lines have been derived from the mouse hybridomas SP2/O―Ag14, NSO and the rat hybridoma YB2/3.0―Ag20. These cell lines illustrate the ability of viral promoters to drive the expression of eukaryotic genes in hybridoma and myeloma cell lines.

The mouse hybridoma cell line SP2/O―Ag14 was chosen as it represents a good candidate for a production cell line as it grows well in serum free medium as a suspension. The transfection of this cell line with plasmids pAC1, pAC2, pAC6 and pPRI demonstrated the ability to obtain cell lines expressing tPA from SP2/O―Ag14. The results with pAC1, pAC2, pAC6 and pPRI demonstrate the potential of the MMT, RSV LTR and Mo MLV LTR promoters to drive expression of eukaryotic proteins in this cell line.

The results for SP2/O―Ag14 (Maximum Levels) were as follows:

| Plasmid | Promoter | Maximum U/ml of Culture |
|---|---|---|
| pAC2 | MMT | 4U/ml |
| pAC1 | Mo MLV LTR | 50U/ml |
| PAC6 | RSV LTR | approx. 10U/ml |
| pPRI | RSV LTR | 4U/ml |

The other mouse hybridoma cell line, NSO, also has the desirable growth characters of SP2/O―Ag14. One plasmid vector (p6GD) was transfected into NSO to generate cell lines. These cell lines were demonstrated to synthesise tPA up to 27U/ml under the influence of the SV40 late promoter.

The rat hybridoma studied was the YB2/3.0―Ag20 cell line which has proved valuable in generating high producing hybridoma cell lines. This cell line was productively transfected with pAC1, pAC6, pPRI, P6GD, pRSD3 and p6 gpt demonstrating the ability of the Mo MLV LTR, RSV LTR and the SV40 late promoters to drive expression in this cell line.

The results for the cell lines obtained by transfection are shown below:

| YB2/3.0―Ag20 (Maximum Levels) | | |
|---|---|---|
| Plasmid | Promoter | Maximum Units/ml of Culture |
| pAC1 | Mo MLV LTR | 50 |
| pAC6 | RSV LTR | 200 |
| pPRI | RSV LTR | 900 |
| p6GD | SV40 late | +Ve |
| p6 gpt | SV40 late | 6.6 |
| pRSD3 | RSV LTR | 45 |

### Amplification

The amplification of the gene copy number is a recognised route to improve the production of protein from expression systems. This has been largely due to the recognition of gene amplification as a mechanism naturally found in various specialised tissues or species (Schimke, 1984). This mechanism has been utilised in eukaryotic expression systems but only significantly in Chinese hamster cell lines (Kaufman *et al*, 1983). The best characterised amplification system is that based on dhfr and methotrexate (Schimke, 1984). Thus with this background it was of interest to see if co-amplification of gene expression could be made to function usefully in the mouse and rat hybridoma/myeloma cell lines.

The study carried out made use of the plasmid construct pRSD3 and the cell line YB2/3.0―Ag20. This plasmid vector was transfected into YB2/3.0―Ag20 and clones selected using 150 nM methothrexate. These cell lines were screened and producing clones were selected to grow on higher and higher levels of methotrexate using recognised methods (Kaufman, *et al*, 1983). The cell lines were stored at the start of the amplification and at intervals during the amplification. The range of expression for tPA was typical with a wide spectrum of activity being observed in the initial cell lines isolated.

Our results demonstrated that as resistance to methotrexate increased so did the level of tPA production demonstrating that indeed co-amplification with hybridoma/myeloma cell lines was valuable.

This utility is best illustrated with our best example cell line YO/pRSD3/2D. This started at 45U/ml tPA growing in 150 nM methotrexate. The level of tPA production was raised to 5800U/ml growing in 3 »M methotrexate after 5 months.

In order to prove the value of these transfected cells in large scale culture, one cell line was selected for evaluation in a small airlift fermenter. The cell line chosen was YB2/3.0Ag20 transfected with pPRI, a clone which had demonstrated high levels of production in stationary culture, pRI 1/10.

### Fed Batch Culture of Cell line pPRI 1/10 in an Airlift Fermenter

The aim of this experiment was to assess growth and tPA synthesis by cell line pPRI 1/10 in a production type cell culture reactor.

The cell reactor used was an aiflift fermenter (ALF) of 5 litres working volume with automatic control of dissolved oxygen tension (DOT), pH and temperature. DOT was controlled by regulated injection of air or oxygen into a sparged ballast gas of nitrogen or air pH was controlled by regulated injection of carbon dioxide into this gas mixture or by applying a pumped feed of alkali to the culture. Temperature was controlled by a flow of temperature regulated water to the reactor jacket.

Cell stocks of the cell line pPRI 1/10 were routinely grown in roller bottle culture in a growth medium consisting of Dulbecco's modification of Eagle's medium (DMEM) supplemented with heat-inactivated foetal calf serum (FCS) at 10% vol/vol.

Medium for the ALF culture was a serum-free formulation consisting of a DMEM base supplemented with albumin, insulin, transferrin, ethanolamine, choline, vitamins, trace metals and a shear protective polymer.

Cell inoculum for the ALF culture was grown in serum-supplemented medium. The cells were sedimented by centrifugation and resuspended in the serum-free growth medium prior to inoculation into the ALF. During the ALF culture supplementing nutrients were added. These consisted of:
a) a shot addition of glutamine to give an increase of 2 mM final concentration in the culture
b) a shot addition of the "insoluble" amino acids tryptophan, tyrosine and cysteine
c) a pumped feed consisting of glucose choline and the "soluble" amino acids.

Samples were removed from the culture at daily intervals. Cell counts were performed using a modified Fuchs Rosenthal counting chamber and cell viability was assessed by exclusion of Trypan Blue stain. Aliquots of culture supernatant were snap frozen in liquid nitrogen and then stored at -70°C for subsequent quantitation of tPA by tPA ELISA and by fibrin agar plate assay.

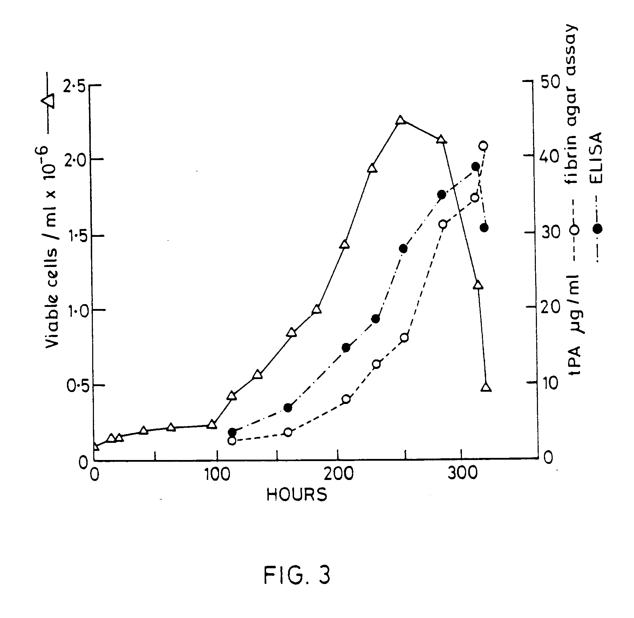

Figure 3 shows profiles for growth and tPA synthesis by pPRI 1/10 cells in airlift culture. Cells attained a maximum viable population density of 2.2 × 10⁶/ml and a maximum total population density of 4 × 10⁶/ml. tPA was synthesised throughout the duration of the culture, reaching a maximum concentration of 42 »g/ml measured by fibrin agar plate assay and 38 »g/ml measured by tPA ELISA. The close agreement between ELISA (which would detect both active and inactive tPA) and the fibrin agar plate assay indicates that the tPA synthesised was fully active.

The invention is described by way of example only, and modifications of detail may be made within the scope of the invention.

### References

Southern, P. J. and Berg. P., Transformation of mammalian cells to antibiotic resistance with a bacterial gene under control of the SV40 Early Region Promoter, J. Mol. Applied Genetics, 1982; 1:327―341.

Subramani, S., Mulligan, R. and Berg, P. Expression of the mouse dihydrofolate reductase complementary deoxyribonucleic acid in Simian virus 40 vectors. Mol. Cell. Biol. 1981; 1: 854―864.

Mulligan, R. C., and Berg, P. Selection for animal cells that express the Escherichia Coli gene coding for xanthine-guanine phophoriboxyltransferase. Proc. Nat. Acad. Sci. USA, 1981, 78: 2072―2076.

Banerji, J., Olson, L. and Schaffner, W.A. Lymphocyte specific cellular enhancer is located downstream of the joining region in immunoglobulin heavy chain genes. Cell (1983) 33: 729―740.

Graham, F.L., and A. J. van der Eb. A new technique for the assay of infectivity of human adenovirus 5 DNA. Virology (1983) 52: 456―467.

Potter, H., Weir. L., and Leder, P. Enhancer-dependent expression of human R immunoglubulin genes introduced into mouse pre-B₂ lymphocytes by electroporation. Proc. Natl. Acad. Sci USA (1984) 81: 7161―7165.

Lopata, M. A., Cleveland, D. W., and Sollner-Webb, B. High level transient expression of a chloramphenicol acetyl transferase gene by DEME-dextran mediated DNA transfection coupled with a dimethyl sulfoxide or glycerol shock treatment. Nucleic Acids Res. (1984) 12: 5707―5717.

Luthman, H. and Magnusson, G. High efficiency polyoma DNA transfection of chloroquinine treated cells Nucleic Acids Res. (1983) 11: 1295―1308.

Page, M. J., Expression of amplified human beta interferon genes using heavy metal induction in Chinese hamster ovary cells. Gene (1985) 37: 139―144.

Jones, P., Benedict, W., Strickland, S. and Reich, E. (1975). Fibrin overlay methods for the detection of single transformed cells and colonies of transformed cells. Cell 5: 323―329.

Kenten, J. H., Wood, C. R., Stephens, P. E., Bendig, M. M., Boss, M. A. and Hentschel, C. C. A sensitive non-destructive assay for transfected genes. DNA (1986) June, (in Press).

Bos, E. S., Vander Doden, A. A., Van Roog, N. and Schmurs, A. H. W. M. (1981) J. Immunoassay 2, 187―204.

Twigg, A. and Sherratt, D. (1980) Trans-complementable copy-number mutants of plasmid Co1E1. Nature 283: 216―218.

Maniatis, T., Fritsch, E. F. and Sambrook, J. (1982) Molecular cloning (a laboratory manual), Cold Spring Harbor Laboratory.

Pennica, D., Holmes, W. E., Kohr, W. J. Harkins, R. N., Vehar, G. A. Ward, C. A., Bennett W. F., Yelverton, E., Seeburg, P. H., Heyneker, H. L., Goeddel, D. V. and Collen D. (1983). Cloning and expression of human tissue-type plasminogen activator cDNA in *E. coli*. Nature 301: 214―221.

Bothwell, A. L. M., Paskind, M., Reth, M., Imanishi-Kari, T., Rajewsky, K. and Baltimore, D. (1981). Heavy chain variable region contribution to NP^{b} family of antibodies:Somotic mutation evident in 2b. Cell 24: 625―637.

Gillies, S. D., Morrison, S. L., Oi, V. T. and Tonegawa, S. (1983). A tissue-specific transcription enhancer element is located in the major intron of rearranged immunoglobulin heavy chain gene. Cell 33: 717―728.

Lusky, M., and M. Botchan. 1981. Inhibition of SV40 replication in simian cells by specific pBR322 DNA sequences. Nature 293: 79―81.

Hoffmann, J. W., Steffen, D., Gusella, J., Tabin, C., Bird, S., Cowing, D., and Weinberg R. A., 1982. DNA methylation affecting the expression of murine leukemia proviruses. J. Virol 44: 144―157.

Hamer, D., and Walling, M (1982). Regulation *in vivo* of a cloned mammalian gene; cadmium induces the transcription of a mouse metallothionein gene in SV40 vectors. J. Mol. Appl. Genet. 1: 273―288.

Harris, T. J., Patel, J., Stephens, P., Kenten, J., Marston, F. A., Little, S., Emtage, J. S., Opdenakker, G., Volckaert, G., Rombauts, U., Billau, A., and De Somer, P. 1986. Cloning of cDNA coding for human tissue plasminogen activator and its expression in recombinant prokaryotic and eukaryotic cells.

Glanville, N., Durnam D. M., and Palmiter, R. D., 1981. Structure of mouse metallothionein-I gene and its mRNA. Nature 292: 267―269.

Gorman, C. M., Merlino, G. T., Wilingham M. C., Pastan, I and Howard, B. M., 1982. The Rous Sarcoma virus long terminal repeat is a strong promoter when introduced into a variety of eukaryotic cells by DNA-mediated transfection. Proc. Natl. Acad. Sci. USA 79: 6777―6781.

Kohler, G., Howe, S. C., and Milstein, C. (1976). Eur. J. Immunol. 6: 292.

Schimke, R. T. Gene amplification in cultured animal cells (1984) Cell 37: 705―713.

Kaufman, R. J., Wasley, L. C., Spiliotes, A. J., Gossels, S. D., Latt, S. A., Larsen, G. A. and Kay, R. M. (1983) Mol. Cell. Biol. 5: 1750―1759.

Morrison, S. L., and Oi, V. T., Transfer and expression of immunoglobulin genes (1984) Ann. Rev. Immunol. 2: 239―256.

The cell-lines indicated at page 5 of the specification of this application are on deposit at the American Type Culture Collection, 12301 Parklawn Drive, Rockville, Maryland 20852, United States of America.

The deposition details are as follows:

| Accession Number | Date of Deposition |
|---|---|
| ATCC CRL 1581 | 22nd October, 1980 |
| ATCC CRL 1662 | 19th March 1982 |
| TIB 18 | 10th November, 1981 |

The cell-lines were not deposited by the applicants and no representation is hereby made that the cell-lines are available as of right to the public by virtue of the depositions.

## Claims

1. A myeloma cell-line transformed with a vector including a gene coding for a eucaryotic polypeptide and a viral promoter, such that expression occurs of the gene coaing for the eucaryotic polypeptide, directed by the viral promoter, such that the eucaryotic polypeptide is produced at a level greater than 1 mg/L.

2. A myeloma cell-line transformed with a vector including a gene coding for a eucaryotic polypeptide and a mouse metallothionein promoter, such that expression occurs of the gene coding for the eucaryotic polypeptide, directed by the mouse metallothionein promoter.

3. A myeloma cell-line according to claim 1 wherein the promoter is an SV40 promoter.

4. A myeloma cell-line according to claim 3 wherein the promoter is the SV40 late promoter.

5. A myeloma cell-line according to claim 1 wherein the promoter is a long terminal repeat derived from a retrovirus.

6. A myeloma cell-line according to claim 5 wherein the promoter is the Rolls sarcoma virus long terminal repeat.

7. A myeloma cell-line according to claim 5 wherein the promoter is the Moloney murine leukaemia virus long terminal repeat.

8. A myeloma cell-line according to claim 1 or 2 wherein the myeloma cell-line is a rat cell-line.

9. A myeloma cell-line according to claim 8 wherein the myeloma cell-line is the rat YB2/3.0 Ag20 hybridoma cell-line.

10. A myeloma cell-line according to claim 8 or 9 when dependent from claim 1 wherein the promoter is the Rous sarcoma virus long terminal repeat.

11. A myeloma cell-line according to claim 1 or 2 wherein the myeloma cell-line is a mouse cell-line.

12. A myeloma cell-line according to claim 10 wherein the myeloma cell-line is the mouse SP2-OAg hybridoma cell-line, the mouse NSO hybridoma cell-line or an NSO-derived hybridoma cell-line.

13. A myeloma cell-line according to claim 11 or 12 when dependent from claim 1 wherein the promoter is the SV40 late promoter.

14. A process for preparing a eucaryotic polypeptide comprising culturing myeloma cells transformed with a vector including a gene coding for the eucaryotic polypeptide and a viral promoter, such that when the vector is transformed into myeloma cells, expression occurs of the gene coding for the eucaryotic polypeptide, directed by the viral promoter, such that the eucaryotic polypeptide is produced at a level greater than 1 mg/L.

15. A process for preparing a eucaryotic polypeptide comprising culturing myeloma cells transformed with a vector including a gene coding for the eucaryotic polypeptide and a mouse metallothionein promoter, such that when the vector is transformed into myeloma cells, expression occurs of the gene coding for the eucaryotic polypeptide, directed by the mouse metallothionein promoter.

## Patentansprüche

1. Myelom-Zellinie, die mit einem Vektor transformiert ist, der ein für ein eukaryotisches Polypeptid codierendes Gen und einen viralen Promotor enthält, so daß die Expression des für das eukaryotische Polypeptid codierenden Gens durch den viralen Promotor gesteuert wird, wobei das eukaryotische Polypeptid auf einem Level größer als 1 mg/L hergestellt wird.

2. Myelom-Zellinie, die mit einem Vektor transformiert ist, der ein für ein eukaryotisches Polypeptid codierendes Gen und einen Maus-Metallothionein-Promotor enthält, so daß die Expression des für das eukaryotische Polypeptid codierenden Gens durch den Maus-Metallothionein-Promotor gesteuert wird.

3. Myelom-Zellinie nach Anspruch 1, in welcher der Promotor ein SV40 Promotor ist.

4. Myelom-Zellinie nach Anspruch 3, in welcher der Promotor der späte SV40 Promotor ist.

5. Myelom-Zellinie nach Anspruch 1, in welcher der Promotor eine von einem Retrovirus stammende lange terminale Wiederholung ist.

6. Myelom-Zellinie nach Anspruch 5, in welcher der Promotor die lange terminale Wiederholung des Rous-Sarcom-Virus ist.

7. Myelom-Zellinie nach Anspruch 5, in welcher der Promotor die lange terminale Wiederholung des Moloney-Mäuseleukämie-Virus ist.

8. Myelom-Zellinie nach Anspruch 1 oder 2, in welcher die Myelom-Zellinie eine Ratten-Zellinie ist.

9. Myelom-Zellinie nach Anspruch 8, in welcher die Myelom-Zellinie die Ratten YB2/3.0 Ag20 Hybridom-Zellinie ist.

10. Myelom-Zellinie nach Anspruch 8 oder 9, falls von Anspruch 1 abhängig, in welcher der Promotor die lange terminale Wiederholung des Rous-Sarcom-Virus ist.

11. Myelom-Zellinie nach Anspruch 1 oder 2, in welcher die Myelom-Zellinie eine Maus-Zellinie ist.

12. Myelom-Zellinie nach Anspruch 10, in welcher die Myelom-Zellinie die Maus SP2-OAg Hybridom-Zellinie, die Maus NSO-Hybridom-Zellinie oder eine von NSO abgeleitete Hybridom-Zellinie ist.

13. Myelom-Zellinie nach Anspruch 11 oder 12, falls von Anspruch 1 abhängig, in welcher der Promotor der späte SV40 Promotor ist.

14. Verfahren zur Herstellung eines eukaryotischen Polypeptids, umfassend die Kultivierung von Myelomzellen, die mit einem Vektor transformiert sind, der ein für das eukaryotische Polypeptid codierendes Gen und einen viralen Promotor enthält, so daß, wenn der Vektor in die Myelomzellen transformiert ist, die Expression des für das eukaryotische Polypeptid codierenden Gens durch den viralen Promotor gesteuert wird, wobei das eukaryotische Polypeptid auf einem Level größer als 1 mg/L hergestellt wird.

15. Verfahren zur Herstellung eines eukaryotischen Polypeptids, umfassend die Kultivierung von Myelomzellen, die mit einem Vektor transformiert sind, der ein für das eukaryotische Polypeptid codierendes Gen und einen Maus-Metallothionein-Promotor enthält, so daß, wenn der Vektor in die Myelomzellen transformiert ist, die Expression des für das eukaryotische Polypeptid codierenden Gens durch den Maus-Metallothionein-Promotorgesteuert wird.

## Revendications

1. Lignée de cellules de myélome transformée avec un vecteur comprenant un gène codant pour un polypeptide eucaryote et un promoteur viral, de manière que se produise une expression du gène codant pour le polypeptide eucaryote, sous la direction du promoteur viral, de manière que le polypeptide eucaryote soit produit à un niveau supérieur à 1 mg/l.

2. Lignée de cellules de myélome transformée avec un vecteur comprenant un gène codant pour un polypeptide eucaryote et un promoteur de métallothionéine de souris, de manière que se produise une expression du gène codant pour le polypeptide eucaryote, sous la direction du promoteur de métallothionéine de souris.

3. Lignée de cellules de myélome selon la revendication 1, dans laquelle le promoteur est un promoteur SV4O.

4. Lignée de cellules de myélome selon la revendication 3, dans laquelle le promoteur est un promoteur SV4O tardif.

5. Lignée de cellules de myélome selon la revendication 1, dans laquelle le promoteur est une longue répétition terminale provenant d'un rétrovirus.

6. Lignée de cellules de myélome selon la revendication 5, dans laquelle le promoteur est la longue répétition terminale du virus de sarcome de Rous.

7. Lignée de cellules de myélome selon la revendication 5, dans laquelle le promoteur est la longue répétition terminale du virus de la leucémie murine de Moloney.

8. Lignée de cellules de myélome selon la revendication 1 ou 2, dans laquelle la lignée de cellules de myélome est une lignée de cellules de rat.

9. Lignée de cellules de myélome selon la revendication 8, dans laquelle la lignée de cellules de myélome est la lignée de cellules d'hybridome YB2/3.O Ag2O de rat.

10. Lignée de cellules de myélome selon la revendication 8 ou 9, dans laquelle le promoteur est la longue répétition terminale de sarcome de Rous.

11. Lignée de cellules de myélome selon la revendication 1 ou 2, dans laquelle la lignée de cellules de myélome est une lignée de cellules de souris.

12. Lignée de cellules de myélome selon la revendication 10, dans laquelle la lignée de cellules de myélome est la lignée de cellules d'hybridome SP2-OAg de souris, la lignée de cellules d'hybridome NSO de souris ou une lignée de cellules d'hybridome provenant de NSO.

13. Lignée de cellules d'hybridome selon la revendication 11 ou 12, quand elle dépend de la revendication 1, dans laquelle le promoteur est le promoteur tardif SV4O.

14. Procédé de préparation d'un polypeptide eucaryote, comprenant la culture de cellules de myélome transformées avec un vecteur comportant un gène codant pour le polypeptide eucaryote et un promoteur viral, de sorte que, lorsque le vecteur est transformé dans des cellules de myélome, il se produit une expression du gène codant pour le polypeptide eucaryote, sous la direction du promoteur viral, de manière que le polypeptide eucaryote est produit à un niveau supérieur à 1 mg/l.

15. Procédé de préparation d'un polypeptide eucaryote comprenant la culture de cellules de myélome transformées avec un vecteur comportant un gène codant pour le polypeptide eucaryote et un promoteur de métallothionéine de souris, de sorte que, lorsque le vecteur est transformé dans des cellules de myélome, il se produit une expression du gène codant pour le polypeptide eucaryote, sous la direction du promoteur de métallothionéine de souris.
